# NEUE EUROPÄISCHE PATENTSCHRIFT

(11) **EP 2 799 035 B2**
(45) Veröffentlichungstag und Bekanntmachung des Hinweises auf die Entscheidung über den Einspruch: **13.05.2026**
(45) Hinweis auf die Patenterteilung: 23.09.2020
(21) Anmeldenummer: 14178708.5
(22) Anmeldetag: 05.03.2008
(51) Int. Cl.: A61F 2/90

(54) **Implantat zur Beeinflussung des Blutflusses**
Implant for influencing blood flow
Implant pour influer sur la circulation du sang

(30) Priorität: 06.03.2007 DE 102007011219; 14.03.2007 DE 102007012964
(43) Veröffentlichungstag der Anmeldung: 05.11.2014
(62) Teilanmeldung aus: 08716257.4
(73) Patentinhaber: Phenox GmbH, 44801 Bochum (DE)
(72) Erfinder: Hannes, Ralf, 44137 Dortmund (DE); Monstadt, Hermann, 44797 Bochum (DE); Schneider, Manuel, 45529 Hattingen (DE)
(74) Vertreter: Schneiders & Behrendt Bochum

(56) Entgegenhaltungen:
- EP-A1- 2 165 684
- WO-A2-00/44308
- WO-A2-03/043527
- WO-A2-2005/115118
- WO-A2-2006/126182
- DE-A1- 102007 061 931
- US-A- 3 868 956
- US-A- 4 655 771
- US-A- 4 681 110
- US-A- 5 201 757
- US-A- 5 709 713
- US-A1- 2002 165 597
- US-A1- 2002 165 601
- US-B1- 7 008 446

## Beschreibung

Die Erfindung betrifft eine Kombination aus einem Implantat für Blutgefäße und einem Führungsdraht, wobei das Implantat eine Wandung aus einzelnen Filamenten aufweist, die zu einem Rundgeflecht zusammengefasst sind. Das Implantat ist insbesondere dazu bestimmt, den Blutfluss im Bereich von arteriovenösen Fehlbildungen, etwa Fisteln und Aneurysmen, zu beeinflussen. Es kann ferner auch zur Behandlung des ischämischen Schlaganfalls eingesetzt werden, etwa zur Wiederherstellung, Erhöhung oder Aufrechterhaltung des Blutflusses. Das Implantat kann rückholbar ausgelegt sein.

Arteriovenöse Fehlbildungen können in einem Patienten zu erheblichen Beeinträchtigungen und Gefährdungen bis hin zum Tode führen. Dies gilt insbesondere auch für arteriovenöse Fisteln und Aneurysmen, insbesondere dann, wenn sie im zerebralen Bereich auftreten. In der Regel versucht man derartige Fehlbildungen durch Implantate zu verschließen. Derartige Implantate werden in der Regel auf endovaskulärem Weg mit Hilfe von Kathetern gesetzt.

Insbesondere bei Aneurysmen hat sich die Implantierung von Platinspiralen bewährt, die das Aneurysma mehr oder weniger vollständig ausfüllen, den Bluteinstrom weitgehend blockieren und dazu führen, dass sich ein lokaler Thrombus ausbildet, der das Aneurysma ausfüllt und letztlich verschließt. Diese Behandlungsmethode ist allerdings nur bei Aneurysmen geeignet, die über einen relativ engen Zugang zum Gefäßsystem verfügen, sogenannte Beerenaneurysmen. Bei Aussackungen von Blutgefäßen, die über einen weiten Zugang zum Gefäß verfügen, drohen die implantierten Spiralen wieder ausgeschwemmt zu werden und Schäden in anderen Bereichen des Gefäßsystems herbeizuführen.

In solchen Fällen wurde bereits vorgeschlagen, eine Art Stent zu setzen, der die Öffnung des Aneurysmas "vergittert" und dadurch die Ausschwemmung der Okklusionsspiralen verhindert. Derartige Stents, die über eine relativ weitmaschige Wandung verfügen, haben aber eine Reihe von Nachteilen.

Zum einen ist dies die weitmaschige Struktur, die den Blutzutritt in das Aneurysma unbeeinträchtigt zulässt. Ist das Aneurysma aber nicht hinreichend mit dem Okklusionsmittel ausgefüllt, bleibt der Druck auf die Gefäßwandung ungemindert bestehen. Eine Nachbehandlung ist aber unter diesen Umständen nur schwer möglich, da der Stent den Zugang zum Aneurysma beeinträchtigt und die Einbringung weiterer Okklusionsmittel behindert.

Ein weiterer Nachteil ist die fehlende Anpassbarkeit des Stents an seinen Einsatzort. Für eine optimale Funktion sollte sich der Stent dicht an die Gefäßwandung anlegen, ohne jedoch einen übermäßigen Druck auf die Wandung auszuüben. Im Gegensatz zu Stents, die eine Aufweitung des Gefäßes bei Stenosen bewirken sollen, sind diese Stents eher als eine Art Manschette zu verstehen, die das Gefäßlumen und die Endothelwand des Gefäßes möglichst wenig beeinflussen soll. In der Folge sind diese Stents, selbst wenn sie für den Einsatzzweck speziell ausgewählt wurden, nur eingeschränkt an die Anforderungen angepasst.

Aus Drahtgeflecht bestehende Stents sind insbesondere für den Einsatz im koronaren Bereich seit langem bekannt. Diese Stents werden in der Regel als Rundgeflecht gefertigt, wobei die einzelnen Drahtfilamente in gegenläufigen spiralförmigen bzw. helixförmigen Lagen die Stentwandung ausbilden. Es entsteht ein Maschengeflecht, das in radialer Richtung sowohl abstützt als auch für Blut durchlässig ist.

Ein Problem dieser als Rundgeflecht ausgebildeten Stents sind die an den freien Enden bestehenden losen Enden, die, aufgrund ihres geringen Durchmessers, traumatisch wirken können.

Gemäß US-A-4 655 771 (Wallsten) wird ein solcher als Rundgeflecht ausgebildeter Stent in seinen Endbereichen durch U-förmige Verbindungsglieder zwischen den losen Enden atraumatisch gestaltet. Die U-förmigen Verbindungsglieder führen allerdings dazu, dass Spannungen entstehen, die zu einer Deformierung des Stents führen.

Gemäß US-A-5 061 275 (Wallsten et al.) werden die losen Enden derartiger Drahtstents durch Laserbehandlung rundgeschmolzen, um so einer Traumatisierung entgegenzuwirken. Der dort beschriebene Stent besteht ebenfalls aus einem Rundgeflecht, bei dem die einzelnen Drähte im Bereich der Knoten Einprägungen aufweisen, um eine spannungsfreie Fixierung innerhalb der Wandung zu ermöglichen. US 2002/165601 zeigt eine selbstexpandierenden, geflochtenen Stent.

Derartige als Rundgeflecht aus Filamenten bestehende Stents werden, wenn sie zur Behandlung von Stenosen eingesetzt werden, mit Hilfe eines Ballons am Einsatzort hydraulisch ausgeweitet und an der Gefäßwandung fixiert. Während der Einbringung dient der an einem Führungsdraht befestigte Ballon als Transportvehikel, auf das der Stent aufgekrimpt ist. Ein solches Transportvehikel sollte aber für Implantate, die zur Beeinflussung bzw. Kanalisierung des Blutflusses im zerebralen Bereich dienen, nicht eingesetzt werden; vielmehr wäre hier ein Implantat, das sich selbständig an den Gefäßdurchmesser anpasst und an die Gefäßwandung anlegt, von Vorteil.

Ein weiteres Problem der aus Drahtgeflecht bestehenden Stents oder Implantate ist die Fertigung. Vorteilhaft ist die Fertigung als geflochtener Endlosschlauch, der auf die gewünschte Dimension abgelängt wird. Hierbei entstehen im Bereich der beiden Enden des abgelängten Schlauches lose Drahtenden, die aufwendig entschärft werden müssen, beispielsweise durch die oben erwähnten Verbindungsglieder.

Dementsprechend liegt der Erfindung die Aufgabe zugrunde, eine Kombination aus einem Implantat, das den Blutfluss in einem Gefäß beeinflussen kann, dergestalt, dass Aneurysmen soweit als möglich vom Blutfluss abgeschottet werden können, und einem Führungsdraht bereitzustellen. Das Implantat soll, bei optimaler Auswahl von Implantatdurchmesser zu Gefäßdurchmesser, dabei in der Lage sein, sich an den jeweiligen Gefäßdurchmesser anzupassen. Im Bereich von Erweiterungen und Aussackungen soll es maximal seinen Nenndurchmesser annehmen.

Der Erfindung liegt weiterhin die Aufgabe zugrunde, eine Kombination aus einem Implantat und einem Führungsdraht bereitzustellen, wobei das Implantat auf eine atraumatische Art und Weise platziert werden kann, d. h. ohne die Hilfe eines Ballons. Eine solche Platzierungsvorrichtung muß das Implantat bis zur endgültigen Freisetzung aus dem Katheter zuverlässig festhalten und insbesondere auch das Zurückziehen des Implantates in den Katheter ermöglichen, solange noch keine vollständige Freisetzung erfolgt ist.

Diese Aufgabe wird mit einer Kombination aus einem Implantat und einem Führungsdraht gemäß Anspruch 1 gelöst.

Als Material für das Implantat werden solche mit Formgedächtniseigenschaften, beispielsweise Nitinol, verwendet.

In der folgenden Beschreibung wird unter den Begriffen proximales Ende bzw. distales Ende des Rundgeflechts das jeweilige Ende verstanden, dass dem behandelnden Arzt zugewandt bzw. abgewandt ist. Entsprechend läßt sich proximal und distal als dem Führungsdraht des Platzierungssystems zugewandt bzw. abgewandt verstehen.

Die Implantate werden im Nachstehenden anhand eines Rundgeflechts zur Abschottung eines Aneurysmas beschrieben.

Bei den Implantaten handelt es sich insbesondere um keine Stents im eigentlichen Sinne, da sie keine Stützfunktion ausüben. Sie dienen nicht zur Stabilisierung der Gefäßwand sondern zur Kanalisierung des Blutflusses im Bereich der Aneurysmen. Sie sollen beispielsweise auch in einem Aneurysma platzierte Okklusionsmittel daran hindern, in die Gefäßbahn ausgeschwemmt zu werden. Es handelt sich um eine Art Inliner, innere Manschetten oder auch Flow Diverter.

Die Implantate werden als Rundgeflecht aus einer Mehrzahl von Filamenten gefertigt, wobei das Rundgeflecht im Prinzip einen Endlosschlauch bildet. Die jeweils benötigte Implantatlänge wird dann aus diesem Endlosschlauch abgelängt. Die einzelnen Filamente sind dazu spiral- oder helixförmig aufgewickelt, wobei die einzelnen Filamente als Flechtwerk, d. h. einander kreuzend untereinander und übereinander eingebracht werden. Die einzelnen Filamentfäden sind dabei in der Regel in zwei einander in einem konstanten Winkel kreuzenden Richtungen gewickelt, die sich beispielsweise in einem Winkel von 90° schneiden. Erfindungsgemäß bevorzugt sind - im spannungsfreien Normalzustand - Winkel von mehr als 90°, insbesondere von 90 bis 160°, wobei die zu den Enden des Implantats hin offenen Winkel gemeint sind. Eine solche steile Wicklung der Einzelfilamente kann, wenn sie hinreichend dicht ist, zu einem Rundgeflecht mit hoher Oberflächendichte führen, das bei axialer Streckung zu erheblich geringeren Durchmessern auseinandergezogen werden kann. Bei Wegfall der Streckkräfte und hinreichender Rückstellkraft des Filamentmaterials nähert sich das Rundgeflecht dem Nenndurchmesser, d. h. dem ursprünglich spannungsfreien Zustand, wieder an und dehnt sich aus, was zu einer engen Anschmiegung an die Gefäßwand am Implantationsort und zu einer Verdichtung der Maschenstruktur an der Wandung führt. Dies gilt insbesondere auch im Bereich von Gefäßerweiterungen.

Insbesondere werden in einem derartigen Rundgeflecht die an den Implantatenden herausstehenden Filamentenden zumindest paarweise zusammengeführt und miteinander dauerhaft verbunden. Dies kann beispielsweise durch Verschweißen erfolgen, aber auch durch mechanisches Verklammern und Verkleben. Dabei oder zusätzlich werden die verbundenen Filamentenden atraumatisch umgeformt. Am proximalen Ende des Rundgeflechts ist die zumindest paarweise Zusammenführung und dauerhafte Verbindung der Filamentenden zwingend.

Die Implantate werden in der Regel nicht mit Hilfe eines Ballons hydraulisch geweitet und platziert. Gleichwohl ist es notwendig, die Implantate mit einem Führungsdraht so zu verbinden, dass sie zuverlässig geführt werden können. Dies erfolgt erfindungsgemäß über Verbindungselemente, die mit einem Halteelement des zur Platzierung erforderlichen Führungsdrahtes zusammenwirken. Als solche Verbindungselemente sind die miteinander verbundenen Filamentenden des Rundgeflechts vorgesehen.

Gefäßabzweigungen (Bifurkationen) können bei den Implantaten beispielsweise durch Bereiche einer geringeren Maschendichte berücksichtigt werden.

Das Rundgeflecht kann im Prinzip auf jede bekannte Art und Weise geflochten werden, liegt aber insbesondere mehrflechtig vor. Besonders bevorzugt ist eine 2-flechtige Ausführung. Eine enge Flechtigkeit führt insbesondere bei einem dichten Flechtwerk zu einer hohen Beanspruchung der einzelnen Filamente. Insoweit ist die mehrflechtige Ausführung geeignet, Spannung aus dem Flechtwerk zu nehmen, wobei aber eine zu hohe Flechtigkeit zu einem schlechten Verbund im Rundgeflecht führt.

Die Filamente können insbesondere auch mehrfach gefacht sein. Besonders bevorzugt ist eine 2- oder 3-Fachung, bei der jeweils zwei oder drei Filamente parallel laufen. Da bei der Fertigung des Rundgeflechts die Filamente von Spulen zugeführt werden, bedeutet dies, dass von der entsprechenden Spule zwei bzw. drei Filamente gleichzeitig dem Dorn, auf dem das Rundgeflecht gefertigt wird, zugeführt werden.

Erfindungsgemäß sind die Filamentenden insbesondere paarweise miteinander verbunden, wobei bei Mehrfachfilamenten paarweise bedeutet, dass jeweils zwei Bündel aus mehreren Filamenten zusammengeführt werden. Am proximalen Ende des Rundgeflechts ist diese zumindest paarweise Zusammenführung zwingend. Diese Bündelung kann dabei kompakt erfolgen, dergestalt, dass alle Drähte zu einem im Wesentlichen runden Bündel zusammengefasst werden und die Stirnseiten aller Drähte gemeinsam aufgeschmolzen werden, so dass eine einheitliche Kuppel entsteht. Dadurch wird eine stoffschlüssige Verbindung der Drähte untereinander hergestellt und das Bündelende atraumatisch hergerichtet.

Alternativ können die Drähte parallel geführt und als Fächer an den Stirnseiten miteinander verschmolzen werden. Der Vorteil dieser Ausführung ist der relativ geringe Durchmesser im Anbindungsbereich, im Vergleich zur Bündelung der Filamente.

Schließlich ist als weitere Variante die Staffelung der einzelnen Filamente möglich, d. h. die Drähte werden versetzt abgelängt. Jeder Draht wird über seine Stirnfläche mit dem danebenliegenden Draht verbunden. Der längste Draht kann dann die Funktion des Verbinders übernehmen. Die Staffelung kann sowohl mit einer gefächerten als auch mit einer kompakten Führung der Einzeldrähte einhergehen.

In jedem Fall werden die miteinander verbundenen Filamentenden als Verbindungselemente zu einem Halteelement ausgestaltet.

Wie vorstehend beschrieben, kommt es bei der spannungsfreien Anordnung der Einzelfilamente im Rundgeflecht darauf an, die Implantatoberfläche möglichst dicht zu gestalten. Da die Flexibilität des Geflechts erhalten bleiben muß, ist eine 100 %ige Oberflächenabdeckung durch die Filamente praktisch nicht möglich. Bevorzugt ist eine Oberflächenabdeckung im Bereich von 30 bis 80 %, vorzugsweise von 40 bis 70 %.

Zur Verbesserung der Oberflächenabdeckung kann das Rundgeflecht mit einer Folie ummantelt werden, beispielsweise aus Teflon, Silikon oder einem anderen körperverträglichen Kunststoff. Zur Erhöhung der Flexibilität und Dehnbarkeit kann eine solche Kunststofffolie geschlitzt sein, wobei die Schlitzanordnung gestaffelt ist und die Längsrichtung der Schlitze entlang der Umfangslinie des Implantates verläuft. Eine solche Folie kann beispielsweise durch Eintauchen des Implantates in ein entsprechendes flüssiges Folienmaterial (Dispersion oder Lösung) und anschließende Einfügung der Schlitze, beispielsweise mit einem Laser erzielt werden. Durch Eintauchen kann beispielsweise auch eine ganze oder teilweise Füllung der Maschen erreicht werden.

Alternativ ist es möglich, die einzelnen Filamente des Implantats durch Eintauchen in eine Kunststoffdispersion oder Lösung mit einem solchen Kunststoff zu ummanteln und dadurch den Filamentquerschnitt zu erhöhen. In diesem Fall bleiben offene Maschen, jedoch wird die Maschengröße deutlich vermindert.

Das Implantat gemäß der Erfindung wird aus einem Material mit Formgedächtniseigenschaften hergestellt. Insbesondere Nitinol kommt in Frage.

Das Implantat kann auf an und für sich bekannte Weise beschichtet sein. Als Beschichtungsmaterialien kommen insbesondere solche in Frage, wie sie für Stents beschrieben sind, etwa mit antiproliferativen, entzündungshemmenden, antithrombogenen, das Einwachsen fördernden und/oder die Anlagerung hindernden Eigenschaften. Bevorzugt ist eine Beschichtung, die das Einwachsen des Implantats und die Neointimabildung fördert. Es kann sinnvoll sein, das Implantat außen derart zu beschichten und innen mit einem Mittel, das die Anhaftung mindert, etwa Heparin oder ein Derivat davon, ASS oder dazu geeignete Oligosaccharide und Chitinderivate. Hier geeignet sind ferner Schichten aus Nano-Partikeln, etwa ultradünnen Schichten aus polymerem SiO₂, die die Anhaftung mindern.

Die Platzierung der Implantate wird in der Praxis unter Röntgenkontrolle erfolgen. Aus diesem Grund sollte das Implantat ein röntgendichtes Markermaterial aufweisen, sofern es nicht aus einem röntgendichten Material selbst gefertigt ist. Solche röntgendichten Materialien sind insbesondere Tantal, Gold, Wolfram und Platinmetalle, etwa Pt-Ir-Legierungen, wobei letztere bevorzugt sind. Solche Marker können beispielsweise als Markerelemente in bekannter Weise an die Filamentenden angeheftet werden, aber auch als Markerfilamente in die Geflechtstruktur des Implantates eingeflochten werden. Die Ummantelung einzelner Filamente mit einer Helix aus Platindraht oder Draht aus Platinlegierungen ist ebenfalls möglich.

Erfindungsgemäß sind die miteinander verbundenen Filamentenden als Verbindungselemente ausgebildet. Dies kann beispielsweise dadurch erfolgen, dass an diesen Verbindungselementen Kugelabschlüsse mit definiertem Durchmesser angeordnet sind, wobei diese durch Umschmelzen mit Hilfe eines Lasers erzeugt werden können. Es können aber auch mit Hilfe eines Lasers Verbinder stoffschlüssig angeschweißt werden. Eine mechanische Verbindungen über Verkrimpen und dergleichen ist ebenfalls möglich.

Ein derartiger Verbinder kann beispielsweise so ausgebildet sein, dass an zusammengeführte miteinander verschweißte Filamentenden ein Kugelverbinder angeschweißt wird, etwa über einen Verbindungsdraht.

Eine Ausgestaltung der Verbindungselemente in anderer als Kugelform ist ebenfalls möglich, beispielsweise in Form von Ankern, Rechtecken oder anderen Formteilen. Die Verbindungselemente funktionieren in jedem Fall nach dem Schlüssel/Schloß-Prinzip, d. h. sie wirken mit einem Halteelement, das peripher entsprechende Ausnehmungen oder Aufnahmen aufweist, zusammen. Solange Halteelement mit angefügtem Implantat in gestreckter und im Durchmesser verkleinerter Form innerhalb eines Katheters geführt werden, werden beide durch die Katheterwand zwangsweise im Verbund gehalten; nach Austritt des Halteelements aus dem Katheter weitet sich das Implantat zu seinem Enddurchmesser auf und befreit sich dadurch aus den Aufnahmen des Halteelements.

Die Fixierung des Implantats in den Ausnehmungen oder Aufnahmen des Halteelements kann auch durch einen separaten Schlauch erfolgen, der formschlüssig über das Halteelement mit eingepassten Verbindungselementen bzw. Verbindern gezogen ist. Der Schlauch wird nach Erreichen der Endposition des Implantats zurückgezogen und setzt damit das Implantat frei. Anschließend können Halteelement mit Führungsdraht, Schlauch und Katheter zurückgezogen werden.

Entsprechend betrifft die Erfindung auch die Kombination aus einem Implantat der vorbezeichneten Art und einem Führungsdraht, an den das Implantat über das Halteelement gekoppelt ist.

Wie schon vorstehend erwähnt, wird die Kombination aus Halteelement und Implantat durch einen endovaskulären Katheter geführt. Dazu ist das Halteelement scheibenförmig ausgebildet und an seiner Peripherie mit Ausnehmungen zur Aufnahme der Verbindungselemente des Implantats versehen. Dabei ist der Durchmesser des Halteelements so bemessen, dass er ohne Weiteres durch einen üblichen Katheter geführt werden kann, die Verbindungselemente aber von der Innenwand der Katheters in ihren Ausnehmungen gehalten werden. Insoweit ist eine kugelförmige Ausbildung der Verbindungselemente von Vorteil, da die Kontaktfläche mit der Innenwandung eines üblichen Katheters und damit die Reibung und der Führungswiderstand gering gehalten werden können.

Gemäß einer bevorzugten Ausführungsform besteht das Halteelement aus zwei zueinander beabstandeten Fixierelementen, die das Implantat zwischen sich gespannt aufnehmen. In diesem Fall weisen beide Fixierelemente die entsprechenden Aufnahmen für die Verbindungselemente des Implantats auf und das Implantat entsprechende Verbindungselemente sowohl an seinem proximalen wie auch an seinem distalen Ende.

Ein entsprechend ausgebildetes Halteelement mit zwei Fixierelementen kann die beiden Fixierelemente in einem definierten Abstand an ein und demselben Führungsdraht aufweisen, wodurch das Implantat bei vorgegebener Länge auch eine definierte Streckung und Spannung erfährt. Auf diese Art und Weise ist sichergestellt, dass keine Überdehnung stattfindet und die Rückstellkräfte nach der Freisetzung im Gefäß voll wirksam werden können. Alternativ ist aber auch eine Fixierung der Fixierelemente an zwei separaten Führungsdrähten möglich, die eine Einstellung oder Streckung des Implantats durch den behandelnden Arzt oder über eine entsprechende Feststellvorrichtung ermöglicht. Der zweite Führungsdraht kann auch als Führungsrohr ausgebildet sein.

Der Erfindung wird durch die nachfolgenden Darstellungen näher erläutert. Es zeigt:
- Figur 1: ein typisches Beispiel eines Rundgeflechts, wie es erfindungsgemäß zum Einsatz kommt;
- Figur 2: Filamente mit einfacher und zweifacher Fachung;
- Figur 3: ein einflechtiges und ein zweiflechtiges Geflecht;
- Figur 4a: die Art und Weise der Zusammenführung der Filamentenden eines Geflechts;
- Figur 4b: zeigt eine zweite Variante zur Verbindung zweier Filamentenden;
- Figur 5a: eine kompakte, gebündelte Zusammenfassung von Filamentenden;
- Figur 5b: eine gefächerte Zusammenfügung von Filamentenden;
- Figur 5c: eine gestaffelte Zusammenfassung von Filamentenden;
- Figur 6: die Verbindung von Führungsdraht und Rundgeflecht über ein Halteelement;
- Figur 7: eine Ausführungsform mit zwei Halteelementen;
- Figur 8: zeigt eine weitere Variante der Fixierung eines Implantats an einem Halteelement mittels eines Schlauches;
- Figur 9: ein Diagramm, das den Zusammenhang zwischen Oberflächendichte und Längenänderung eines Implantats wiedergibt; und
- Figur 10: die Anordnung eines Implantates im Halsbereich eines Aneurysmas.

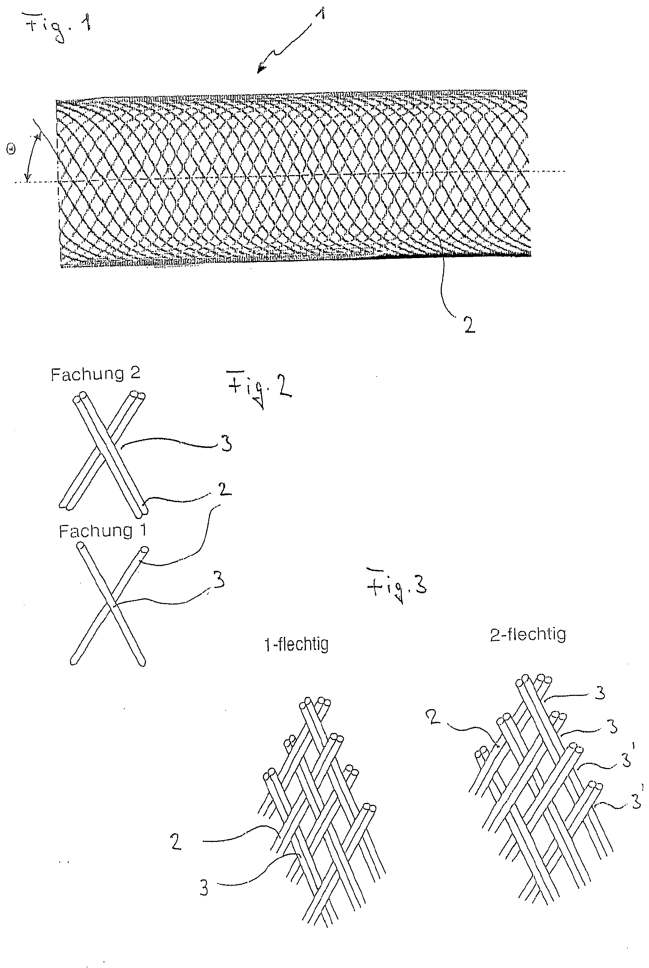

Figur 1 zeigt die Geflechtstruktur eines Implantats 1, das aus miteinander verflochtenen Einzelfilamenten 2 besteht. Die einzelnen Filamente kreuzen sich im gezeigten Fall in einem Winkel von etwa 120°, wobei die offene Seite des Winkels zu den offenen Enden des Rundgeflechts weisen. Das Geflecht ist in leicht gespanntem/gelängtem Zustand dargestellt, d. h. mit einem reduzierten Durchmesser.

Der Winkel Theta bezeichnet den Geflechtswinkel zur Längsachse, der im entspannten Zustand bei dem Nenndurchmesser bis zu 80° betragen kann. Bei der Längung des Geflechts im Katheter kann der Winkel Theta bis auf ca. 7° abnehmen.

Es versteht sich, dass der Nenndurchmesser des Rundgeflechts auf das Lumen des Zielgefäßes an der zu behandelnden Stelle abgestimmt ist.

Das Geflecht wird auf einer konventionellen Flechtmaschine hergestellt und liegt als Endlosgeflecht vor. Geflochten wird auf einem Dorn, dessen äußere Abmessungen dem Innendurchmesser der späteren Produkte entsprechen.

Die Flechtmaschine und deren Besetzung bestimmt die Struktur des Geflechts, z. B. die Anzahl der Fäden, den Fadenverlauf und die Anzahl der Kreuzungspunkte am Umfang und per Schlaglänge. Die Anzahl der Fäden ist abhängig von der Zahl der Klöppel, wobei die Klöppel je zur Hälfte in beide Richtungen um den Flechtkern laufen.

Die Filamente bestehen in der Regel aus Metall, beispielsweise Nitinol. Es können auch Kunststofffäden, die über die nötige Flexibilität verfügen, verwandt werden. Die Filamentstärke beträgt idealerweise 0,01 bis 0,2 mm, insbesondere 0,02 bis 0,1 mm. Um eine hohe Abdeckung der Wandfläche zu erzielen, kann anstelle von Drahtmaterial auch ein Flachbandmaterial verwandt werden, beispielsweise mit einer Breite von 0,05 bis 0,5 mm, vorzugsweise bis 0,1 mm, bei den oben angegebenen Stärken.

Das Rundgeflecht kann aus Einzelfilamenten hergestellten werden (Fachung 1) oder auch aus zwei (Fachung 2) oder mehr Filamenten.

Figur 2 zeigt Knotenpunkte 3, an denen sich jeweils zwei parallel geführte Filamente kreuzen (Fachung 2) oder nur Einzelfilamente 2 kreuzen (Fachung 1). Werden zwei oder mehr Filamente zusammengefasst, werden diese über ein und dieselbe Spule zugeführt.

Figur 3 zeigt Muster für 1-flechtige und 2-flechtige Strukturen aus Filamenten 2 der Fachung 2. In der 1-flechtigen Struktur liegen die Filamentpaare alternierend übereinander und untereinander. Bei der 2-flechtigen Struktur werden die Filamentpaare, wie dargestellt, jeweils über zwei gegenläufige Filamentpaare geführt, bevor sie unter zwei gegenläufigen Filamentpaaren hindurchgeführt werden.

Eine Fachung von zwei oder eine noch höhere Fachung bringt eine höhere Oberflächendichte des Rundgeflechts mit sich, bei gleichzeitiger Verringerung der Längenausdehnung während der Komprimierung des Rundgeflechts. Diese höhere Oberflächendichte geht allerdings auf Kosten der Flexibilität, auch durch Erhöhung der Reibung und Spannung. Dem kann durch eine Erhöhung der Flechtigkeit entgegengewirkt werden, d. h. eine 2- oder höher-flechtige Struktur bringt eine Erhöhung der Flexibilität mit sich. Erfindungsgemäß ist eine Flechtigkeit von 2 und eine Fachung von 2 bevorzugt.

Nach der Auftrennung in für das Produkt spezifische Einheiten muß das Geflecht an den Enden geschlossen werden. Dies ist notwendig, um die Formstabilität des Flechtkörpers zu gewährleisten und um Gefäßverletzungen zu vermeiden. Hierfür ist auch die Ordnung der Struktur an den Enden des Rundgeflechts notwendig.

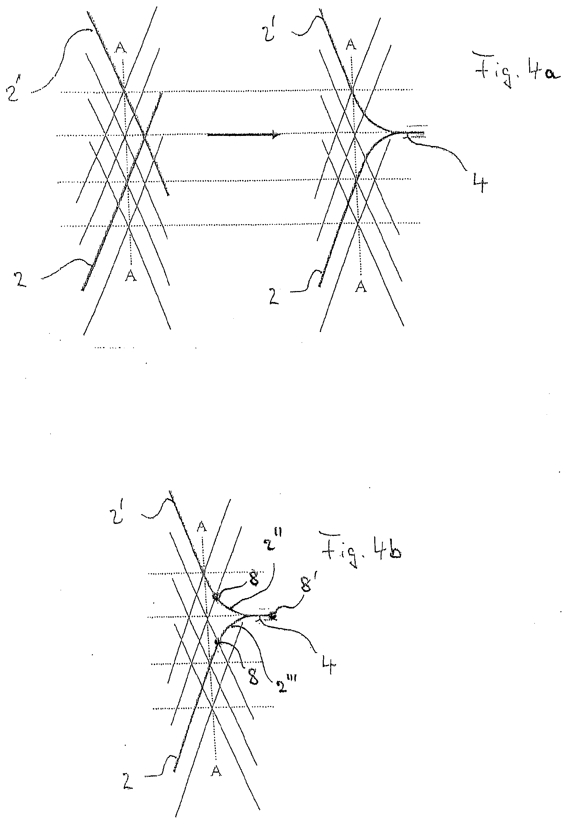

Figur 4a zeigt die Zusammenführung zweier Filamente 2, 2' am Ende des Rundgeflechts zu einem Filamentpaar 4, wobei 2 und 2' gegenläufige Filamente sind. Die Filamente werden dazu in axialer Richtung umgebogen und distal miteinander verschweißt. Dabei werden jeweils die in den randständigen Knoten übereinanderliegenden Filamente miteinander verbunden. Knotenpunkte befinden sich beispielsweise an den Kreuzungspunkten der waagerecht eingezeichneten Hilfslinien mit der senkrechten Knotenebene A-A.

Figur 4b zeigt eine weitere Variante der Zusammenführung zweier Filamente 2 und 2' zu einem Filamentpaar 4, das über zwei Arme 2" und 2'" und Schweißpunkte 8 mit den Filamenten 2, 2' verbunden ist. Die beiden Arme des Verbindungsstücks 4 sind im Schweißpunkt 8' zusammengeführt. Das Filamentpaar 4 kann vorgefertigt werden und ermöglicht einen gleichmäßigen, den Verlauf der Filamente 2, 2' nicht störenden Abschluss.

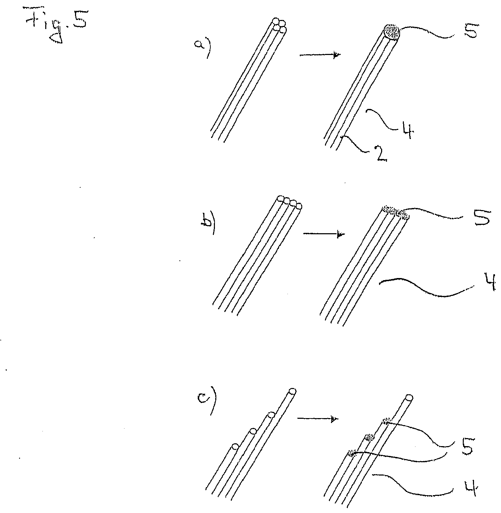

Figur 5 zeigt Varianten für das Schließen des Geflechts, wie in Figur 4 generell beschrieben. Gemäß Figur 5a wird ein Bündel von vier Filamenten 2 stirnseitig miteinander verschweißt, beispielsweise durch Laserverschweißen. Dies führt zum einen zu einer dauerhaften Verbindung der einzelnen Filamente, was die Auflösung des Rundgeflechts verhindert, und bewirkt zugleich eine atraumatische Umgestaltung der ansonsten doch verletzungsträchtigen Filamentspitzen. Gemäß einer bevorzugten Ausführungsform können die Schweißstellen 5 zu einer Kugel oder einem Formkörper umgeschaltet werden, die als Verbindungselemente dienen können.

Figur 5b zeigt eine fächerförmige Führung eines Filamentbündels 4, bei dem Einzelfilamente distal über einen gemeinsamen Schweißpunkt 5 miteinander verbunden sind.

Figur 5c schließlich zeigt ein gestaffeltes Auslaufen der einzelnen Filamente des Filamentbündels 4 mit einzelnen Schweißpunkten 5, die die Verbindung untereinander herstellen.

Bei dieser Variante sind die Drähte versetzt abgelängt. Der längste Draht kann die Funktion des Verbinders übernehmen und beispielsweise ein Formelement an seiner Spitze aufnehmen. Alle kürzeren Drähte werden mit ihrer Stirnfläche mit dem danebenliegenden Draht stoffschlüssig verbunden. Bei dieser Ausführungsform ist der zu erwartende geringere Durchmesser des Anbindungsbereichs von besonderem Interesse.

Bei einer 2-flechtigen Geflechtstruktur der Fachung 2 mit insgesamt 16 Doppelfäden führt dies zu insgesamt acht Schweißverbindungen der in Figur 5 gezeigten Art (andere sind denkbar), die zu acht Verbindungselementen für die Anbindung des Implantats an ein Halteelement dienen können.

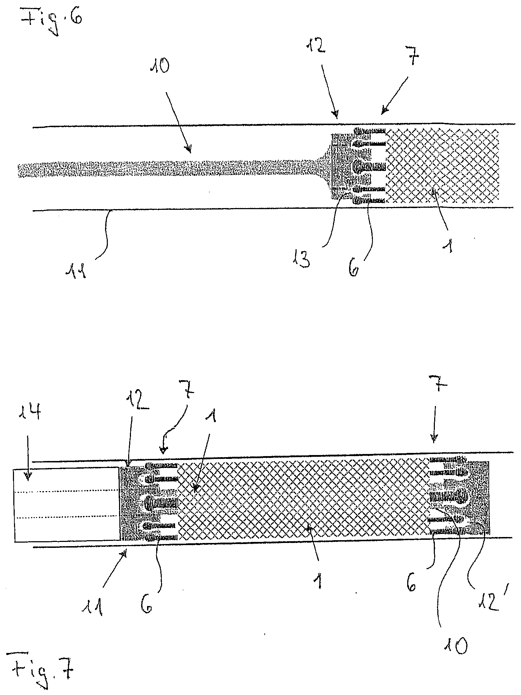

Figur 6 zeigt eine erfindungsgemäße Kombination aus einem Implantat 1, das über ein Halteelement 12 mit einem Führungsdraht 10 verbunden ist und in einem Katheter 11 geführt wird. Das Implantat 1 ist proximal über die endständigen miteinander verschweißten Filamentbündel 7, die zu Verbinder 6 umgeformt sind, mit einem Halteelement 12 verbunden. Das Halteelement 12 weist an seiner Peripherie Ausnehmungen auf, in die die Verbinder 6, die über eine Art kugeligen Kopf verfügen, eingepasst sind. Das Halteelement 12, das einen scheibenförmigen Querschnitt aufweist, ist an die Innenweite des Katheters 11 angepasst, dergestalt, dass die in den Ausnehmungen 13 angeordneten Verbinder 6 durch die innere Katheterwand daran gehindert werden, aus den Ausnehmungen herauszutreten.

Wird das Implantat 1 mit Hilfe des Führungsdrahtes 10 aus dem Ende des Katheters 11 herausgeführt, weitet es sich unter Entspannung auf und nimmt einen größeren Durchmesser an. Dabei treten die Verbinder 6 aus den Ausnehmungen 13 aus, das Implantat ist frei, Führungsdraht und Halteelement 12 können zurückgezogen werden. Das Implantat 1 schmiegt sich bei der Expansion eng an die Gefäßwand an und vermag auf diese Art und Weise eine arteriovenöse Fehlbildung wie ein Aneurysma abzuschirmen.

Figur 7 zeigt eine weitere Variante der in Figur 6 gezeigten Kombination aus Führungsdraht, Halteelement und Implantat in einem Katheter 11. Gemäß dieser Variante ist es möglich, das Implantat zwischen einem proximalen Halteelement 12 und einem distalen Halteelement 12' zu spannen. Beide Halteelemente 12, 12' verfügen über die peripheren Ausnehmungen, in die die endständigen zusammengeschweißten Filamentbündel 7 über die Verbinder 6 eingepasst sind. Das Geflecht 1 dazwischen kann, je nach Abstand der Halteelemente 12, 12' eine größere oder kleinere Längung erfahren, was dazu genutzt werden kann, den Transport durch den Katheter zu erleichtern.

Um die Relativbewegung realisieren zu können, bedarf es zweier autarker Führungssysteme. Dies kann beispielsweise durch zwei separate Führungsdrähte 10 erfolgen. Eine weitere Variante ist die Anbindung des proximalen Halteelements 12 an ein flexibles Rohr und die Anbindung des distalen Halteelements 12' an einen in diesem Rohr verlaufenden Führungsdraht 10. Um die Flexibilität des Systems zu gewährleisten, können Rohr 14 und Führungsdraht 10 aus Nitinol gefertigt sein.

Bevor es zur Freisetzung des Implantats kommt, muß das System distal positioniert werden. Nach der distalen Freisetzung kann zunächst der Führungsdraht 10 mit dem distalen Halteelement 12' in den Katheter 11 zurückgezogen werden. Anschließend kann mit dem Rückzug des Katheters 11 das Implantat 1 auch proximal freigesetzt werden und das proximale Halteelement 12 zusammen mit dem Rohr 14 zurückgezogen werden.

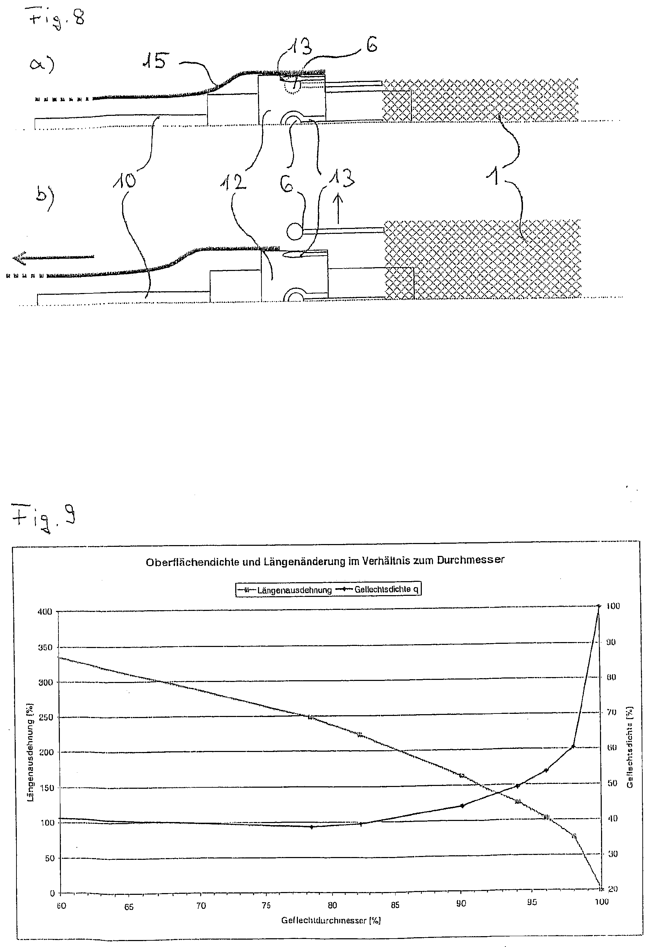

Das Freisetzungssystem gemäß Figur 8a zeigt eine weitere Variante einer erfindungsgemäßen Kombination aus einem Implantat 1, das über ein Halteelement 12 mit einem Führungsdraht 10 verbunden ist und in einem hier nicht dargestellten Katheter geführt wird. Das Implantat 1 ist proximal über die endständig angeschweißten Verbinder 6 mit dem Halteelement 12 verbunden dergestalt, dass die Verbinderkugeln 6 in Ausnehmungen 13 des Halteelements 12 eingepasst sind. Ein über das Halteelement 12 gezogener Schlauch 15, in dem der Führungsdraht 10 verläuft, fixiert die Verbinder 6 in den Ausnehmungen 13.

Figur 8a zeigt die Kombination aus Implantat, Halteelement, Führungsdraht und Schlauch mit fixiertem Implantat, Figur 8b das sich aus dem Halteelement 12 freigesetzte Implantat nach Zurückziehen des Schlauches 15.

Da der Führungsdraht 10 und der Schlauch 15 innerhalb eines nicht dargestellten Katheters relativ zueinander bewegt werden können, ist es möglich, das Implantat zunächst am Implantationsort zu platzieren, danach den Katheter zurückzuziehen und anschließend durch Zurückziehen des Schlauches 15 das Implantat freizusetzen. Schlauch 15 und Führungsdraht 1 mit Halteelement 12 können anschließend in den Katheter zurückgezogen und mit diesem aus dem Gefäß entfernt werden. Ein noch nicht abgelöstes aber distal freigesetztes Implantat kann wieder eingezogen werden um es erneut zu platzieren oder zu entfernen.

Hinreichend zugfeste Schläuche sind an und für sich bekannt. Um die notwendige Flexibilität auch bei längeren Schläuchen zu gewährleisten, kann es sinnvoll sein, den Schlauch mit Schlitzen oder anderen Öffnungen vor allem im distalen Bereich zu versehen. Dies erhöht die Biegsamkeit und gibt die Möglichkeit, vor der Anwendung des Systems die Luft darauf zu verdrängen.

Andere Varianten dieses Freisetzungssystems sind denkbar, bei denen beispielsweise die Verbinderkugeln 6 durch anders geartete Verbindungselemente ersetzt werden und durch den Katheter 11, ein Rohr 14 oder einen Schlauch 15 in ihrer Position am Halteelement 12 fixiert werden.

Es versteht sich, dass die hier gezeigte Fixierung eines Implantats an einem Halteelement mit Hilfe eines Rohrs, Schlauchs oder innerhalb des Katheters auch für andere Formen von Implantaten verwendbar ist.

Figur 9 schließlich zeigt ein Diagramm, das den Zusammenhang zwischen Oberflächendichte und Längenänderung eines geflochtenen Stents, wie er Gegenstand der Erfindung ist, wiedergibt.

In voll expandiertem Zustand (Ruhezustand), hat ein Implantat bei einem Geflechtdurchmesser von 100 % eine Längenausdehnung von 0 %. Bei zunehmender Längung nimmt der Geflechtdurchmesser ab und verdoppelt seine Länge bei einer Durchmesserreduktion um 4 % bzw. verdreifacht seine Länge bei einer Durchmesserreduktion von 15 %. Dies bedeutet, dass ein Inliner zum Transport durch einen Mikrokatheter, der in das Blutgefäß nahe am Zielort eingeführt werden muß, erheblich gelängt werden muß.

Parallel dazu erreicht die Geflechtdichte einen Wert von 100 % bei vollständiger Expansion, d. h. einer Längenausdehnung von 0 %. Die Geflechtdichte nimmt bei einer Verminderung des Geflechtdurchmessers von 4 % (Längenausdehnung 100 %) um 46 % ab, bei einem Geflechtdurchmesser von nur noch 85 % auf eine Geflechtdichte von 40 %.

Das Diagramm gibt die theoretischen Werte für ein idealisiertes zylindrisches Geflecht wieder. Zu berücksichtigen ist allerdings, dass Gefäße keine ideale zylindrische Form haben und insbesondere im Bereich eines Aneurysmahalses, den es abzudecken gilt, häufig, auch aufgrund der Öffnung, einen höheren Gefäßdurchmesser im Vergleich zu dem anliegenden gesunden Gefäßabschnitt aufweist. Diese Eigenschaft der Fehlbildung ermöglicht es dem Geflecht des Implantats Inliners, lokal an gewünschter Stelle eine höhere Oberflächendichte anzunehmen und so den Blutfluss in das Aneurysma zu vermindern bzw. zu stoppen.

In der Regel wird sich eine Oberflächendichte/Geflechtdichte von etwa 40 bis 70 % des voll expandierten Zustandes einstellen. Bei gut eingepassten Implantaten oder bei starker Aufweitung des Gefäßdurchmessers im Bereich des Aneurysmas können allerdings auch Werte von deutlich über 70 % des theoretisch möglichen Wertes erzielt werden.

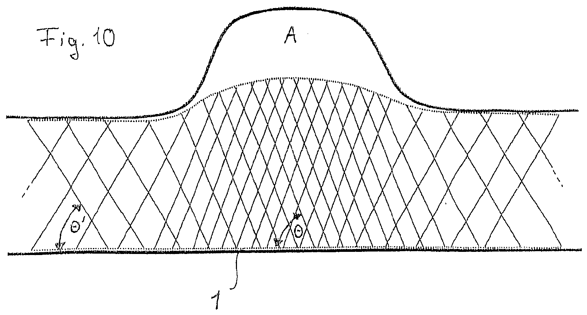

Figur 10 zeigt in einer Prinzipskizze den Sitz eines Implantats in einem Gefäß im Halsbereich eines Aneurysmas A. Das Geflecht 1 hat im Aneurysmabereich A seine größte Expansion erreicht, mit einem Geflechtswinkel Theta von etwa 68°. Entsprechend ist die Filament- oder Geflechtsdichte erhöht. Im Randbereich des Rundgeflechts 1 bei normalem Gefäßlumen beträgt der Geflechtswinkel Theta 60°, bei einer entsprechend reduzierten Filamentdichte.

Durch die im Aneurysmabereich A erhöhte Filamentdichte ist die Durchlässigkeit des Rundgeflechts vermindert, was der Intention entspricht, den Blutfluss im Aneurysmabereich A zu kanalisieren und das Aneurysma A vom Blutfluss "abzuschneiden". Diese Abschottung kann beispielsweise durch Ausfüllen des Aneurysmaraumes A außerhalb des Implantats 1 mit einer Okklusionsspirale oder einem anderen Okklusionsmittel weiter gefördert werden.

## Patentansprüche

1. Kombination aus einem Implantat (1) für Blutgefäße zur Beeinflussung des Blutflusses im Bereich von Aneurysmen (A) und einem Führungsdraht (10), wobei das Implantat (1) über eine Wandung aus einzelnen Filamenten (2, 2') verfügt, die zu einem Rundgeflecht zusammengefasst sind, wobei das Rundgeflecht ein abgelängtes Endlosgeflecht ist, in einem Einführungskatheter (11) eine gelängte Form mit vermindertem Durchmesser annimmt und am Implantatort unter Anpassung an den Gefäßdurchmesser und Zunahme der Oberflächenabdeckung expandiert, wobei die Filamente (2, 2') des Rundgeflechts Formgedächtniseigenschaften haben,
**dadurch gekennzeichnet,**
**dass** das Rundgeflecht in der Weise eingestellt ist, dass es sich bei Wegfall der Streckkräfte, die das Rundgeflecht in die gelängte Form bringen, so stark ausdehnt, dass es im Bereich eines Aneurysmahalses, an dem das Gefäß einen höheren Gefäßdurchmesser im Vergleich zu dem anliegenden gesunden Gefäßabschnitt aufweist, eine höhere Oberflächenabdeckung im Vergleich zu dem anliegenden gesunden Gefäßabschnitt annimmt, und wobei das Implantat (1) über ein Halteelement (12) an den Führungsdraht (10) gekoppelt ist, wobei am proximalen Ende des Rundgeflechts Filamentenden zumindest paarweise zusammengeführt und miteinander dauerhaft verbunden sind, wobei die verbundenen Filamentenden atraumatisch umgeformt und als Verbindungselemente (6) zum Halteelement (12) ausgebildet sind und das Halteelement (12) peripher Ausnehmungen (13) aufweist, in die die Verbindungselemente (6) eingepasst sind.

2. Kombination nach Anspruch 1, **dadurch gekennzeichnet, dass** die Filamente (2, 2') zumindest teilweise aus Nitinol bestehen.

3. Kombination nach Anspruch 1 oder 2, **dadurch gekennzeichnet, dass** die Verbindungselemente (6) an den Filamentenden angeordnete Kugelabschlüsse mit definiertem Durchmesser sind.

4. Kombination nach einem der vorstehenden Ansprüche, **dadurch gekennzeichnet, dass** die Filamentenden miteinander verschweißt sind.

5. Kombination nach einem der vorstehenden Ansprüche, **dadurch gekennzeichnet, dass** die Filamentenden in axialer Richtung zusammengeführt und miteinander verbunden sind.

6. Kombination nach einem der vorstehenden Ansprüche, **dadurch gekennzeichnet, dass** die Geflechtstruktur eine Oberflächenabdeckung durch die Filamente (2, 2') im Bereich von 30 bis 80 % aufweist.

7. Kombination nach Anspruch 6, **dadurch gekennzeichnet, dass** die Oberflächenabdeckung 40 bis 70 % beträgt.

8. Kombination nach einem der vorstehenden Ansprüche, **dadurch gekennzeichnet, dass** die Filamente (2, 2') des Rundgeflechts mit Kunststoff ummantelt sind.

9. Kombination nach einem der vorstehenden Ansprüche, **dadurch gekennzeichnet, dass** das Rundgeflecht mit einer Folie ummantelt ist oder mit Kunststoff ganz oder teilweise gefüllte Maschen aufweist.

10. Kombination nach einem der vorstehenden Ansprüche, **dadurch gekennzeichnet, dass** das Implantat Markerelemente enthält.

11. Kombination nach Anspruch 10, **dadurch gekennzeichnet, dass** die Markerelemente Filamente aus Platin oder Platinlegierungen sind, die in das Rundgeflecht eingeflochten sind oder Filamente (2, 2') des Rundgeflechts helixartig umgeben.

## Claims

1. Combination of an implant (1) for blood vessels for influencing the blood flow in the region of aneurysms (A) and a guide wire (10), the implant (1) having a wall made of individual filaments (2, 2') which are combined to form a round braid, the round braid being a continuous braid cut to length, assuming an elongated shape having a reduced diameter in an introduction catheter (11) and expanding at the implantation site with adaptation to the vessel diameter and an increase in surface coverage, the filaments (2, 2') of the round braid having shape-memory properties,
**characterised in that**
the round braid is set in such a way that, upon cessation of the stretching forces that bring the round braid into the elongated shape, it expands so strongly that, in the region of an aneurysm neck, at which the vessel has a greater vessel diameter in comparison with the adjoining healthy vessel section, it assumes a higher surface coverage in comparison with the adjoining healthy vessel section, and wherein the implant (1) is coupled to the guide wire (10) via a retaining element (12), wherein, at the proximal end of the round braid, filament ends are brought together at least in pairs and are permanently interconnected, wherein the interconnected filament ends are atraumatically reshaped and designed as connecting elements (6) for the retaining element (12), and the retaining element (12) has peripheral recesses (13) into which the connecting elements (6) are fitted.

2. Combination according to claim 1, **characterised in that** the filaments (2, 2') consist at least partially of nitinol.

3. Combination according to claim 1 or 2, **characterised in that** the connecting elements (6) are spherical end pieces arranged on the filament ends and having a defined diameter.

4. Combination according to one of the preceding claims, **characterised in that** the filament ends are welded to one another.

5. Combination according to one of the preceding claims, **characterised in that** the filament ends are brought together and interconnected in the axial direction.

6. Combination according to one of the preceding claims, **characterised in that** the braid structure has a surface coverage by the filaments (2, 2') in the range of 30 to 80%.

7. Combination according to claim 6, **characterised in that** the surface coverage is 40 to 70%.

8. Combination according to one of the preceding claims, **characterised in that** the filaments (2, 2') of the round braid are sheathed with plastics material.

9. Combination according to one of the preceding claims, **characterised in that** the round braid is sheathed with a film or has meshes completely or partially filled with plastics material.

10. Combination according to one of the preceding claims, **characterised in that** the implant contains marker elements.

11. Combination according to claim 10, **characterised in that** the marker elements are filaments of platinum or platinum alloys which are woven into the round braid or helically surround filaments (2, 2') of the round braid.

## Revendications

1. Combinaison d'un implant (1) pour vaisseaux sanguins destiné à influer sur la circulation du sang dans la zone d'anévrismes (A) et d'un fil de guidage (10), l'implant (1) disposant d'une paroi constituée de filaments (2, 2') individuels, qui sont rassemblés en un treillis rond, le treillis rond étant un treillis sans fin coupé à longueur, adoptant, dans un cathéter d'introduction (11), une forme allongée avec un diamètre réduit et se déployant sur le site d'implantation en s'adaptant au diamètre du vaisseau et en augmentant la couverture de surface, les filaments (2, 2') du treillis rond présentant des propriétés de mémoire de forme,
**caractérisée en ce que**
le treillis rond est réglé de telle manière que, lors de la suppression des forces d'étirage qui mettent le treillis rond dans la forme allongée, il se dilate si fortement que, dans la zone d'un collet d'anévrisme, au niveau duquel le vaisseau présente un diamètre vasculaire plus important par comparaison avec la section de vaisseau saine adjacente, il atteint une couverture de surface supérieure par comparaison avec la section de vaisseau saine adjacente, et dans laquelle l'implant (1) est couplé au fil de guidage (10) par un élément de retenue (12), des extrémités de filament étant réunies au moins par paires à l'extrémité proximale du treillis rond et reliées entre elles de manière durable, les extrémités de filament reliées étant déformées de manière atraumatique et réalisées sous la forme d'éléments de liaison (6) avec l'élément de retenue (12), et l'élément de retenue (12) présentant des évidements périphériques (13), dans lesquels les éléments de liaison (6) sont ajustés.

2. Combinaison selon la revendication 1, **caractérisée en ce que** les filaments (2, 2') sont constitués au moins en partie de nitinol.

3. Combinaison selon la revendication 1 ou 2, **caractérisée en ce que** les éléments de liaison (6) sont des terminaisons sphériques disposées sur les extrémités de filament et présentant un diamètre défini.

4. Combinaison selon l'une des revendications précédentes, **caractérisée en ce que** les extrémités de filament sont soudées entre elles.

5. Combinaison selon l'une des revendications précédentes, **caractérisée en ce que** les extrémités de filament sont réunies dans la direction axiale et reliées entre elles.

6. Combinaison selon l'une des revendications précédentes, **caractérisée en ce que** la structure de treillis présente une couverture de surface par les filaments (2, 2') comprise entre 30 et 80 %.

7. Combinaison selon la revendication 6, **caractérisée en ce que** la couverture de surface est de 40 à 70 %.

8. Combinaison selon l'une des revendications précédentes, **caractérisée en ce que** les filaments (2, 2') du treillis rond sont revêtus de matière plastique.

9. Combinaison selon l'une des revendications précédentes, **caractérisée en ce que** le treillis rond est revêtu d'une feuille ou comporte des mailles remplies entièrement ou en partie de matière plastique.

10. Combinaison selon l'une des revendications précédentes, **caractérisée en ce que** l'implant contient des éléments marqueurs.

11. Combinaison selon la revendication 10, **caractérisée en ce que** les éléments marqueurs sont des filaments en platine ou en alliages de platine, qui sont tressés dans le treillis rond ou qui entourent de manière hélicoïdale des filaments (2, 2') du treillis rond.
